# EUROPEAN PATENT APPLICATION

(11) **EP 4 814 122 A1**
(43) Date of publication of application: **30.09.2026**
(21) Application number: 25166407.4
(22) Date of filing: 26.03.2025
(51) Int. Cl.: C12N 15/82

(54) **MODIFIED TOMATO PLANTS**

(71) Applicant: Johann Wolfgang Goethe-Universität Frankfurt am Main, 60323 Frankfurt am Main (DE)
(72) Inventor: Fragkostefanakis, Sotirios, 60438 Frankfurt am Main (DE); Bakery, Ayat, 60438 Frankfurt am Main (DE)
(74) Representative: Kuttenkeuler, David

(57) **Abstract**

The invention is based on an increased expression of heat stress transcription factor B2a (HSFB2a) protein in plants to reduce cleistogamy and thereby increase plant function such as seed or fruit production. In one alternative, the invention is based on a decreased expression of heat stress transcription factor B2a (HSFB2a) protein in plants to enhance, increase and/or promote cleistogamy. The invention provides genetic constructs for the expression of HSFB2a in plants and transgenic plant cells and plants comprising the constructs of the invention.

## Description

### FIELD OF THE INVENTION

The invention is based on an increased expression of heat stress transcription factor B2a (HSFB2a) protein in plants to reduce cleistogamy and thereby increase plant function such as seed or fruit production. The invention provides genetic constructs for the expression of HSFB2a in plants and transgenic plant cells and plants comprising the constructs of the invention. In an alternative the invention pertains to nucleic acids, proteins, methods and uses for promoting, increasing or enhancing cleistogamy in a plant.

### DESCRIPTION

Tomatoes (Solanum lycopersicum) are among the most widely cultivated and consumed horticultural crops worldwide, serving as a staple ingredient in diverse cuisines and providing essential nutrients such as vitamins A and C, lycopene, and antioxidants. Over the years, the development of improved tomato cultivars has played a critical role in enhancing agricultural productivity, nutritional value, and marketability.

Modern breeding techniques, including hybridization, marker-assisted selection, and genome editing, have led to the introduction of tomato varieties with superior traits. These advancements address key agricultural challenges, such as resistance to pests and diseases, tolerance to abiotic stresses (e.g., drought, extreme temperatures, and soil salinity), extended shelf life, and enhanced flavor profiles. Moreover, improvements in yield stability and post-harvest quality contribute significantly to meeting the rising global demand for tomatoes in both fresh and processed forms.

Cleistogamy is a desirable trait in crops like tomatoes as it favors self-pollination and therefore enhance fruiting rates and seed production. Consequently, in many crops cleistogamy is an important agronomic trait. During domestication and breeding, the elimination of genes, namely self-incompatibility (SI) and stigma shortening (mutations in the Style2.1 and SE3.1 genes) have facilitated the transition from anisogamy to cleistogamy (Shang et al, 2021, The Plant Cell, Volume 33, Issue 10, October 2021; Chen et al, Science 26 Oct 2007 Vol 318, Issue 5850). Therefore, in modern tomato cultivars the stigma is shorter and enclosed to the anther cone. However, in many tomato cultivars, exposure to high temperatures leads to the elongation of the style and the exertion of the stigma from the anther cone, leading to pollination failure and lower fruit set.

The present invention relates to a novel tomato cultivar that exhibits a combination of desirable agronomic characteristics, including high yield potential and enhanced stress resilience. This cultivar represents a valuable innovation in commercial tomato production, offering advantages to growers, distributors, and consumers alike.

### BRIEF DESCRIPTION OF THE INVENTION

Generally, and by way of brief description, the main aspects of the present invention can be described as follows:

In **the first aspect,** the invention pertains to a nucleic acid construct comprising a polynucleotide encoding a heat stress transcription factor B2a (HSFB2a) protein; and a heterologous, constitutive- or tissue-specific promoter operably linked to the polynucleotide encoding the HSFB2a polypeptide, wherein the promoter specifically directs expression of the HSFB2a polypeptide in the plant or tissue of the plant. In an alternative of the first aspect, the invention pertains to a nucleic acid construct comprising a polynucleotide encoding a mutated heat stress transcription factor B2a (HSFB2a) protein; and a heterologous, constitutive- or tissue-specific promoter operably linked to the polynucleotide encoding the HSFB2a polypeptide, wherein the promoter specifically directs expression of the mutated HSFB2a polypeptide in the plant or tissue of the plant.

In **the second aspect,** the invention pertains to a recombinant expression vector comprising a nucleic acid construct comprising a polynucleotide encoding a heat stress transcription factor B2a (HSFB2a) protein (or a mutated or inactivated version thereof); and a heterologous, constitutive- or tissue-specific promoter operably linked to the polynucleotide encoding the HSFB2a polypeptide, wherein the promoter specifically directs expression of the HSFB2a polypeptide in the plant or tissue of the plant.

In **the third aspect,** the invention pertains to a recombinant host cell comprising a nucleic acid construct (or recombinant expression vector) comprising a polynucleotide encoding a heat stress transcription factor B2a (HSFB2a) protein (or a mutated or inactivated version thereof); and a heterologous, constitutive- or tissue-specific promoter operably linked to the polynucleotide encoding the HSFB2a polypeptide, wherein the promoter specifically directs expression of the HSFB2a polypeptide in the plant or tissue of the plant.

In **the fourth aspect,** the invention pertains to a recombinant plant or plant part, comprising a genetic modification which enhances the expression of heat stress transcription factor B2a (HSFB2a) - preferably mRNA or protein expression of HSFB2a, in a tissue of the plant. Or alternatively the invention pertains to a recombinant plant or plant part, comprising a genetic modification which decreases the expression of (or decreases the activity of) heat stress transcription factor B2a (HSFB2a) - preferably mRNA or protein expression of HSFB2a, in a tissue of the plant.

**In the fifth aspect,** the invention pertains to recombinant plant parts, such as recombinant plant seeds, fruit, rootstock or cutting, comprising a nucleic acid, recombinant expression vector and/or recombinant cell, according to any of the previous aspects of the invention.

In **the sixth aspect,** the invention pertains to a method of enhancing plant growth or yield comprising: providing a recombinant plant transformed with the nucleic acid construct according to any one of the preceding aspects; and growing the plant under conditions effective to permit the nucleic acid construct to express the HSFB2a polypeptide in the recombinant plant, and thereby enhance plant growth or yield; and/or to a method of enhancing plant growth or yield comprising: providing a recombinant plant seed transformed with the nucleic acid construct according to any one of the preceding aspects; planting the recombinant plant seed in a growth medium; and propagating a recombinant plant from the plant seed to permit the nucleic acid construct to express the HSFB2a polypeptide in the recombinant plant, and thereby enhance plant growth or yield; and/or the invention pertains to a method of enhancing plant growth or yield comprising: providing a rootstock, cutting, or seed according any one of the preceding aspects; introducing the rootstock, cutting, or seed into a growth medium; and propagating a recombinant plant from the rootstock, cutting, or seed to permit the nucleic acid construct to express the HSFB2a polypeptide in the recombinant plant, and thereby enhance plant growth or yield.

In **the seventh aspect,** the invention pertains to a method for alleviating stigma exertion in a plant, the method comprising a step of artificially increasing in the plant an amount, level, concentration and/or activity of
(i) a protein having an amino acid sequence shown in SEQ ID NO: 1, or a protein variant having an amino acid sequence with at least 60% sequence identity compared to the sequence shown in SEQ ID NO: 1; and/or
(ii) a nucleic acid, such as a gene or mRNA, encoding a protein or protein variant of (i).

In **the eighth aspect,** the invention pertains to a method for alleviating stigma exertion in a plant, the method comprising a step of artificially decreasing in the plant an amount, level, concentration and/or activity of
(i) a protein having an amino acid sequence shown in SEQ ID NO: 1, or a protein variant having an amino acid sequence with at least 60% sequence identity compared to the sequence shown in SEQ ID NO: 1; and/or
(ii) a nucleic acid, such as a gene or mRNA, encoding a protein or protein variant of (i).

### DETAILED DESCRIPTION OF THE INVENTION

In the following, the elements of the invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine two or more of the explicitly described embodiments or which combine the one or more of the explicitly described embodiments with any number of the disclosed and/or preferred elements. Furthermore, any permutations and combinations of all described elements in this application should be considered disclosed by the description of the present application unless the context indicates otherwise.

The term "amino acid sequence" means the sequence of amino acids that characterizes a given protein.

The term "polypeptide" means a polymer of amino acids joined together by peptide bonds.

The term "polynucleotide" or "nucleic acid" used in the present disclosure refers to a DNA polymer composed of multiple nucleotides chemically bonded by a series of ester linkages between the phosphor) I group of one nucleotide and the hydroxyl group of the sugar in the adjacent nucleotide.

The polynucleotides described in the present description include "genes" and nucleic acid molecules described including "vectors" or "plasmids". Accordingly, the term "gene", also called a "structural gene" refers to a polynucleotide that codes for a particular sequence of amino acids, which comprise all or part of one or more proteins or enzymes, and may include regulatory (non-transcribed) DNA sequences, such as promoter sequences, which determine for example the conditions under which the gene is expressed.

The term "nucleotide sequence" means the order in which nucleotides are situated in a chain relative to one another.

The term "sequence identity" means the number percentage of matches in positions from an alignment of two molecular sequences.

The term "primer" refers to a single-stranded oligonucleotide, the 3' end of which can be used as the initiation site for the DNA synthesis with a DNA polymerase.

A "vector" is any means by which a nucleic acid can be propagated and/or transferred between organisms, cells or cellular components. Vectors include viruses, bacteriophage, pro-viruses, plasmids, phagemids, transposons and artificial chromosomes such as YACs (yeast artificial chromosomes), BACs (bacterial artificial chromosomes), and PLACs (plant artificial chromosomes), and the like, that are "episomes", that is, that replicate autonomously or can integrate into a chromosome of a host cell. A vector can also be a naked RNA polynucleotide, a polynucleotide composed of both DNA and RNA within the same strand, a poly- lysine-conjugated DNA or RNA, a peptide conjugated DNA or RNA, a liposome- conjugated DNA, or the like, that are not episomal in nature, or it can be an organism which comprises one or more of the above polynucleotide constructs such as Agrobacterium or a bacterium.

The term "recombinant" means a cell or organism in which genetic recombination has occurred. It also includes a molecule (e.g., a nucleic acid or a polypeptide) that has been artificially or synthetically (i.e., non-naturally) altered by human intervention. The alteration can be performed on the molecule within, or removed from, its natural environment or state.

The term "recombinant vector" means a vector carrying a foreign DNA fragment.

The term "recombinant host cell" means a host cell carrying a recombinant vector.

The term "recombinant DNA construct" means a molecule that is constructed outside living cells by joining natural or synthetic DNA to a DNA molecule that can replicate in a living cell.

The term "promoter" refers to a polynucleotide molecule that is in its native or non native state located upstream or 5' to a translational start codon of an open reading frame (or protein-coding region) and that is involved in recognition and binding of RNA polymerase II and other proteins (trans-acting transcription factors) to initiate transcription.

The term "transformation" refers to the process by which a recombinant DNA molecule is introduced into a host cell. Transformation (or transduction, or transfection), can be achieved by any one of a number of means including electroporation, microinjection, biolistics (or particle bombardment-mediated delivery), or Agrobacterium- mediated transformation.

The term "transgenic plant" means plant that has been genetically engineered to artificially introduce a gene or set of gene sequences in the plant genome.

A transgenic "plant cell" means a plant cell that is transformed with stably- integrated, non-natural, recombinant polynucleotides, e.g. by Agrobacterium- mediated transformation or by bombardment using micro particles coated with recombinant polynucleotides.

The term "expression" with respect to a gene sequence refers to transcription of the gene and, as appropriate, translation of the resulting mRNA transcript to a protein. Thus, as will be clear from the context, expression of a protein results from transcription and translation of the open reading frame sequence.

The term "heterologous" refers to a transfected DNA part, that is not of the host's autonomous DNA or simply isn't a part of the host's genetic material. The term "heterologous promoter" used herein refers to the constitutive promoter obtained from the other source than the host per se.

In **the first aspect,** the invention pertains to a nucleic acid construct comprising a polynucleotide encoding a heat stress transcription factor B2a (HSFB2a) protein; and a heterologous, constitutive- or tissue-specific promoter operably linked to the polynucleotide encoding the HSFB2a polypeptide, wherein the promoter specifically directs expression of the HSFB2a polypeptide in the plant or tissue of the plant. In an alternative of the first aspect, the invention pertains to a nucleic acid construct comprising a polynucleotide encoding a mutated heat stress transcription factor B2a (HSFB2a) protein; and a heterologous, constitutive- or tissue-specific promoter operably linked to the polynucleotide encoding the HSFB2a polypeptide, wherein the promoter specifically directs expression of the mutated HSFB2a polypeptide in the plant or tissue of the plant.

Heat Stress Transcription Factor B2a (HSFB2a) is a key regulatory gene in Solanum lycopersicum that plays a critical role in the plant's response to heat stress. As a member of the HSF (Heat Stress Factor) family, HSFB2a functions as a transcriptional regulator involved in modulating the expression of heat-responsive genes. Unlike classical activator-type HSFs (HSFA members), HSFB2a primarily acts as a co-regulator, fine-tuning heat stress responses to balance growth and stress adaptation.

The HSFB2a protein contains a conserved DNA-binding domain that allows it to interact with heat shock elements (HSEs) in the promoter regions of target genes. It has been shown to participate in heat stress signal transduction, influencing cellular homeostasis and proteostasis by regulating heat shock proteins (HSPs) and other protective mechanisms. Recent studies suggest that HSFB2a contributes to maintaining plant development under high-temperature conditions by repressing or modulating excessive stress responses, preventing unnecessary energy expenditure.

The HSFB2a protein amino acid sequence is provided herein below as SEQ ID NO: 1 or encoded by a nucleic acid sequence shown in SEQ ID NO: 3.

The **HSFB2a** molecules (such as nucleic acids or proteins) can be part of a vector, such as a plasmid vector not found in plants. In one example, such a vector has at least 80%, at least 90%, at least 95%, at least 96%, at least 97%, at least 98%, at least 99%, or 100% sequence identity SEQ ID NO: 1 to 3. Also provided are isolated transgenic plant cells, transgenic plant parts, and transgenic plants which include the **HSFB2a** molecules, such as a vector including an **HSFB2a** molecule. The disclosed transgenic plant cells, transgenic plant parts, and transgenic plants can further include one or more additional exogenous nucleic acid(s) encoding a protein(s) that confers upon the transgenic plant, transgenic plant part, or transgenic plant cell a desired trait, wherein the desired trait is one or more of herbicide tolerance, drought tolerance, heat tolerance, low or high soil pH level tolerance, salt tolerance, resistance to an insect, resistance to a bacterial disease, resistance to a viral disease, resistance to a fungal disease, resistance to a nematode, resistance to a pest, male sterility, site-specific recombination; abiotic stress tolerance, modified phosphorus characteristics, modified antioxidant characteristics; modified essential seed amino acid characteristics, decreased phytate, modified fatty acid metabolism, and modified carbohydrate metabolism. The disclosed transgenic plant cells, transgenic plant parts, and transgenic plants can further include single locus conversion. Exemplary plant parts include a protoplast, leaf, stem, root, root tips, anther, pistil, stamen, seed, embryo, pollen, ovule, microspore, protoplast, sporophyte, gametophyte, cotyledon, hypocotyl, flower, shoot, tissue, petiole, or meristematic cell. In some examples, the transgenic plant cells, transgenic plant parts, and transgenic plants is or is from a dicot.

In an embodiment of the invention the nucleic acid construct further comprises a 3' transcription termination polynucleotide.

In another embodiment of the invention, the nucleic acid construct comprises DNA.

Another embodiment of the invention pertains nucleic acid constructs wherein the promoter is a constitutive expression promoter, such as Cauliflower Mosaic Virus 35S (PCaMV35S) promoter.

In another embodiment, the nucleic acid construct comprises a CaMV35S terminator sequence (TCaMV35S).

In another embodiment, the nucleic acid construct comprises a sequence shown in SEQ ID NO: 3.

In **the second aspect,** the invention pertains to a recombinant expression vector comprising a nucleic acid construct comprising a polynucleotide encoding a heat stress transcription factor B2a (HSFB2a) protein (or a mutated or inactivated version thereof); and a heterologous, constitutive- or tissue-specific promoter operably linked to the polynucleotide encoding the HSFB2a polypeptide, wherein the promoter specifically directs expression of the HSFB2a polypeptide in the plant or tissue of the plant.

Suitable promoters which can be used with the nucleic acid of the present disclosure include constitutive, inducible, or tissue-specific promoters.

Suitable constitutive promoters include, for example, CaMV 35S promoter (Odell et al., Nature 313:810-812, 1985); maize Ubi 1 (Christensen et al., Plant Sol. Biol. 18:675-689, 1992); rice actin (McElroy et al., Plant Cell 2: 163-171, 1990); pEMU (Last et al., Theor. Appl. Genet. 81:581-588, 1991); and Synthetic Super MAS (Ni et al., The Plant Journal 7: 661-76, 1995). Other constitutive promoters include those in U.S. Pat. Nos. 5,659,026, 5,608,149; 5,608,144; 5,604,121; 5,569,597: 5,466,785; 5,399,680; 5,268,463; and 5,608,142.

Suitable inducible promoters can be pathogen-inducible promoters such as, for example, the alfalfa PR10 promoter (Coutos-Thevenot et al., Journal of Experimental Botany 52: 901-910, 2001 and the promoters described by Marineau et al., Plant Mol. Biol. 9:335- 342, 1987; Matton et al. Molecular Plant-Microbe Interactions 2:325-331, 1989; Somsisch et al., Proc. Natl. Acad. Sci. USA 83:2427-2430, 1986: Somsisch et al., Mol. Gen. Genet. 2:93- 98, 1988; and Yang, Proc. Natl. Acad. Sci. USA 93: 14972-14977, 1996.

Suitable tissue-specific promoters include, but not limited to, leaf-specific promoters such as described, for example, by Yamamoto et al., Plant J. 12:255-265, 1997; Kwon et al., Plant Physiol. 105:357-67, 1994; Yamamoto et al., Plant Cell Physiol. 35:773-778, 1994; Gotor et al., Plant J. 3:509-18, 1993; Orozco et al., Plant Mol. Biol. 23: 1129-1138, 1993; and Matsuoka et al., Proc. Natl. Acad. Sci. USA 90:9586-9590, 1993.

The nucleic acid construct of the present disclosure may also include at least one selectable marker such as, for example, nptll. Preferably, the nucleic acid construct is a shuttle vector, which can propagate both in E. coli (wherein the construct comprises an appropriate selectable marker and origin of replication) and be compatible for propagation in cells. The construct according to the present disclosure can be, for example, a plasmid, a bacmid, a phagemid, a cosmid, a phage, a virus or an artificial chromosome, preferably a plasmid.

The nucleic acid construct of the present disclosure can be utilized to stably transform plant cells. The principle methods of causing stable integration of exogenous DNA into plant genome include two main approaches: (i) Agrobacterium-mediated gene transfer: Klee et al. (1987) Annu. Rev. Plant Physiol. 38:467-486; Klee and Rogers in Cell Culture and Somatic Cell Genetics of Plants, Vol. 6, Molecular Biology of Plant Nuclear Genes, eds. Schell, J., and Vasil, L. K., Academic Publishers, San Diego, Calif. (1989) p. 2-25; Gatenby, in Plant Biotechnology, eds. Kung, S. and Amtzen, C. J., Butterworth Publishers, Boston, Mass. (1989) p. 93-112. (ii) Direct DNA uptake: Paszkowski et al., in Cell Culture and Somatic Cell Genetics of Plants, Vol. 6, Molecular Biology of Plant Nuclear Genes eds. Schell, J., and Vasil, L. K., Academic Publishers, San Diego, Calif. (1989) p. 52-68; including methods for direct uptake of DNA into protoplasts, Toriyama, K. et al. (1988) Bio/Technology 6: 1072-1074. DNA uptake induced by brief electric shock of plant cells: Zhang et al. Plant Cell Rep. (1988) 7:379-384. Fromm et al. Nature (1986) 319:791-793. DNA injection into plant cells or tissues by particle bombardment, Klein et al. Bio/Technology (1988) 6:559-563; McCabe et al. Bio/Technology (1988) 6:923-926; Sanford, Physiol. Plant. (1990) 79:206-209; by the use of micropipette systems: Neuhaus et al., Theor. Appl. Genet. (1987) 75:30-36; Neuhaus and Spangenberg, Physiol. Plant. (1990) 79:213-217; glass fibers or silicon carbide whisker transformation of cell cultures, embryos or callus tissue, U.S. Pat. No. 5,464,765 or by the direct incubation of DNA with germinating pollen, DeWet et al. in Experimental Manipulation of Ovule Tissue, eds. Chapman, G. P. and Mantell, S. H. and Daniels, W. Longman, London, (1985) p. 197-209; and Ohta, Proc. Natl. Acad. Sci. USA (1986) 83:715- 719.

The Agrobacterium system includes the use of plasmid vectors that contain defined DNA segments that integrate into the plant genomic DNA. Methods of inoculation of the plant tissue vary depending upon the plant species and the Agrobacterium delivery system. A widely used approach is the leaf disc procedure which can be performed with any tissue explant that provides a good source for initiation of whole plant differentiation. Horsch et al. in Plant Molecular Biology Manual A5, Kluwer Academic Publishers, Dordrecht (1988) p. 1- 9. A supplementary approach employs the Agrobacterium delivery system in combination with vacuum infiltration. Suitable Agrobacterium-mediated procedures for introducing exogenous DNA to plant cells is described by Dougale et al. (Journal of General Virology, 79:2301-2311, 1998) and in U.S. Pat. No. 6,395,962.

There are various methods of direct DNA transfer into plant cells. In electroporation, the protoplasts are briefly exposed to a strong electric field. In microinjection, the DNA is mechanically injected directly into the cells using very small micropipettes. In microparticle bombardment, the DNA is adsorbed on microprojectiles such as magnesium sulfate crystals or tungsten particles, and the microprojectiles are physically accelerated into cells or plant tissues.

Alternatively, the nucleic acid construct of the present disclosure can be introduced into plant cells by a microprojectiles bombardment. In this technique, tungsten or gold particles coated with exogenous DNA are accelerated toward the target cells. Suitable plant transformation procedures by microprojectiles bombardment are described by Sagi et al. (Biotechnology 13:481-485, 1995) and by Dougale et al. (Journal of General Virology, 79:2301-2311, 1998). Preferably, the nucleic acid construct of the present disclosure is introduced into plant cells by a microprojectiles bombardment procedure as described in Example 4 herein below.

**In the third aspect,** the invention pertains to a recombinant host cell comprising a nucleic acid construct (or recombinant expression vector) comprising a polynucleotide encoding a heat stress transcription factor B2a (HSFB2a) protein (or a mutated or inactivated version thereof); and a heterologous, constitutive- or tissue-specific promoter operably linked to the polynucleotide encoding the HSFB2a polypeptide, wherein the promoter specifically directs expression of the HSFB2a polypeptide in the plant or tissue of the plant.

In **the fourth aspect,** the invention pertains to a recombinant plant or plant part, comprising a genetic modification which enhances the expression of heat stress transcription factor B2a (HSFB2a) - preferably mRNA or protein expression of HSFB2a, in a tissue of the plant. Or alternatively the invention pertains to a recombinant plant or plant part, comprising a genetic modification which decreases the expression of (or decreases the activity of) heat stress transcription factor B2a (HSFB2a) - preferably mRNA or protein expression of HSFB2a, in a tissue of the plant.

The disclosed transgenic plants that include one or more disclosed transgenic nucleic acid molecules having a promoter operably linked to an HSFB2a coding sequence, in some examples express at least about 10%, at least 20%, at least 25%, at least 30%, at least 40%, at least 50%, at least 60%, at least 70%, at least 80%, at least 90%, at least 100%, at least 200%, at least 300%, at least 400% or at least 500% greater levels (such as at least 10-fold, at least 25-fold, at least 100-fold, at least 250-fold, at least 750-fold, or at least 1000-fold more) of HSFB2a in plant cells as compared to a wild-type plant of the same species.

Asa Methods of producing transgenic plants are well known to those of ordinary skill in the art. Transgenic plants can now be produced by a variety of different transformation methods including, but not limited to, electroporation; microinjection; microprojectile bombardment, also known as particle acceleration or biolistic bombardment; viral-mediated transformation; and Agrobacterium-mediated transformation. See, for example, U.S. Patent Nos. 5,405,765; 5,472,869; 5,538,877; 5,538,880; 5,550,318; 5,641,664; 5,736,369 and 5,736,369; International Patent Application Publication Nos. WO2002/038779 and WO/2009/117555; Lu et ak, (Plant Cell Reports, 2008, 27:273-278); Watson et ak, Recombinant DNA, Scientific American Books (1992); Hinchee et ak, Bio/Tech. 6:915-922 (1988); McCabe et ak, Bio/Tech. 6:923-926 (1988); Toriyama et ak, Bio/Tech. 6:1072-1074 (1988); Fromm et ak, Bio/Tech. 8:833-839 (1990); Mullins et ak, Bio/Tech. 8:833-839 (1990); Hiei et ak, Plant Molecular Biology 35:205-218 (1997); Ishida et ak, Nature Biotechnology 14:745-750 (1996); Zhang et ak, Molecular Biotechnology 8:223-231 (1997); Ku et ak, Nature Biotechnology 17:76-80 (1999); and, Raineri et ak, Bio/Tech. 8:33-38 (1990)), each of which is expressly incorporated herein by reference in their entirety.

Agrobacterium tumefaciens is a naturally occurring bacterium that is capable of inserting its DNA (genetic information) into plants, resulting in a type of injury to the plant known as crown gall. Most species of plants can now be transformed using this method, including cucurbitaceous species.

Microprojectile bombardment is also known as particle acceleration, biolistic bombardment, and the gene gun (Biolistic^{®} Gene Gun). The gene gun is used to shoot pellets that are coated with genes (e.g., for desired traits) into plant seeds or plant tissues in order to get the plant cells to then express the new genes. The gene gun uses an actual explosive (.22 caliber blank) to propel the material. Compressed air or steam may also be used as the propellant. The Biolistic^{®} Gene Gun was invented in 1983-1984 at Cornell University by John Sanford, Edward Wolf, and Nelson Allen. It and its registered trademark are now owned by E. I. du Pont de Nemours and Company. Most species of plants have been transformed using this method.

The most common method for the introduction of new genetic material into a plant genome involves the use of living cells of the bacterial pathogen Agrobacterium tumefaciens to literally inject a piece of DNA, called transfer or T-DNA, into individual plant cells (usually following wounding of the tissue) where it is targeted to the plant nucleus for chromosomal integration. There are numerous patents governing Agrobacterium mediated transformation and particular DNA delivery plasmids designed specifically for use with Agrobacterium- for example, US4536475, EP0265556, EP0270822, WO8504899, WO8603516, US5591616, EP0604662, EP0672752, WO8603776, WO9209696, WO9419930, WO9967357, US4399216, WO8303259, US5731179, EP068730, WO9516031, US5693512, US6051757 and EP904362A1.

Agrobacterium-mediated plant transformation involves as a first step the placement of DNA fragments cloned on plasmids into living Agrobacterium cells, which are then subsequently used for transformation into individual plant cells. Agrobacterium-mediated plant transformation is thus an indirect plant transformation method. Methods of Agrobacterium-mediated plant transformation that involve using vectors with no T-DNA are also well known to those skilled in the art and can have applicability in the present disclosure. See, for example, U.S. Patent No. 7,250,554, which utilizes P-DNA instead of T-DNA in the transformation vector.

A transgenic plant formed using Agrobacterium transformation methods typically contains a single gene on one chromosome, although multiple copies are possible. Such transgenic plants can be referred to as being hemizygous for the added gene. A more accurate name for such a plant is an independent segregant, because each transformed plant represents a unique T-DNA integration event (U.S. Patent No. 6,156,953). A transgene locus is generally characterized by the presence and/or absence of the transgene. A heterozygous genotype in which one allele corresponds to the absence of the transgene is also designated hemizygous (U.S. Patent No. 6,008,437).

Direct plant transformation methods using DNA have also been reported. The first of these to be reported historically is electroporation, which utilizes an electrical current applied to a solution containing plant cells (M. E. Fromm et ah, Nature, 319, 791 (1986); H. Jones et ah, Plant Mol. Biol., 13, 501 (1989) and H. Yang et ak, Plant Cell Reports, 7, 421 (1988). Another direct method, called "biolistic bombardment", uses ultrafine particles, usually tungsten or gold, that are coated with DNA and then sprayed onto the surface of a plant tissue with sufficient force to cause the particles to penetrate plant cells, including the thick cell wall, membrane and nuclear envelope, but without killing at least some of them (US 5,204,253, US 5,015,580). A third direct method uses fibrous forms of metal or ceramic consisting of sharp, porous or hollow needle-like projections that literally impale the cells, and also the nuclear envelope of cells. Both silicon carbide and aluminum borate whiskers have been used for plant transformation (Mizuno et al., 2004; Petolino et al., 2000; US5302523 US Application 20040197909) and also for bacterial and animal transformation (Kaepler et al., 1992; Raloff, 1990; Wang, 1995). There are other methods reported, and undoubtedly, additional methods will be developed. However, the efficiencies of each of these indirect or direct methods in introducing foreign DNA into plant cells are invariably extremely low, making it necessary to use some method for selection of only those cells that have been transformed, and further, allowing growth and regeneration into plants of only those cells that have been transformed.

For efficient plant transformation, a selection method must be employed such that whole plants are regenerated from a single transformed cell and every cell of the transformed plant carries the DNA of interest. These methods can employ positive selection, whereby a foreign gene is supplied to a plant cell that allows it to utilize a substrate present in the medium that it otherwise could not use, such as mannose or xylose (for example, refer US 5767378; US 5994629). More typically, however, negative selection is used because it is more efficient, utilizing selective agents such as herbicides or antibiotics that either kill or inhibit the growth of non-transformed plant cells and reducing the possibility of chimeras. Resistance genes that are effective against negative selective agents are provided on the introduced foreign DNA used for the plant transformation. For example, one of the most popular selective agents used is the antibiotic kanamycin, together with the resistance gene neomycin phosphotransferase (nptll), which confers resistance to kanamycin and related antibiotics (see, for example, Messing & Vierra, Gene 19: 259-268 (1982); Bevan et al., Nature 304:184-187 (1983)). However, many different antibiotics and antibiotic resistance genes can be used for transformation purposes (refer US 5034322, US 6174724 and US 6255560). In addition, several herbicides and herbicide resistance genes have been used for transformation purposes, including the bar gene, which confers resistance to the herbicide phosphinothricin (White et al., Nucl Acids Res 18: 1062 (1990), Spencer et al., Theor Appl Genet 79: 625-631(1990), US 4795855, US 5378824 and US 6107549). In addition, the dhfr gene, which confers resistance to the anticancer agent methotrexate, has been used for selection (Bourouis et al., EMBO J. 2(7): 1099-1104 (1983).

The expression control elements used to regulate the expression of a given protein can either be the expression control element that is normally found associated with the coding sequence (homologous expression element) or can be a heterologous expression control element. A variety of homologous and heterologous expression control elements are known in the art and can readily be used to make expression units for use in the present disclosure. Transcription initiation regions, for example, can include any of the various opine initiation regions, such as octopine, mannopine, nopaline and the like that are found in the Ti plasmids of Agrobacterium tumefaciens. Alternatively, plant viral promoters can also be used, such as the cauliflower mosaic virus 19S and 35S promoters (CaMV 19S and CaMV 35S promoters, respectively) to control gene expression in a plant (U.S. Patent Nos. 5,352,605; 5,530,196 and 5,858,742 for example). Enhancer sequences derived from the CaMV can also be utilized (U.S. Patent Nos. 5,164,316; 5,196,525; 5,322,938; 5,530,196; 5,352,605; 5,359,142; and 5,858,742 for example). Lastly, plant promoters such as prolifera promoter, fruit specific promoters, Ap3 promoter, heat shock promoters, seed specific promoters, etc. can also be used.

Either a gamete -specific promoter, a constitutive promoter (such as the CaMV or Nos promoter), an organ-specific promoter (such as the E8 promoter from tomato), or an inducible promoter is typically ligated to the protein or antisense encoding region using standard techniques known in the art. The expression unit may be further optimized by employing supplemental elements such as transcription terminators and/or enhancer elements.

Thus, for expression in plants, the expression units will typically contain, in addition to the protein sequence, a plant promoter region, a transcription initiation site and a transcription termination sequence. Unique restriction enzyme sites at the 5' and 3' ends of the expression unit are typically included to allow for easy insertion into a pre-existing vector.

In the construction of heterologous promoter/structural gene or antisense combinations, the promoter is preferably positioned about the same distance from the heterologous transcription start site as it is from the transcription start site in its natural setting. As is known in the art, however, some variation in this distance can be accommodated without loss of promoter function.

In addition to a promoter sequence, the expression cassette can also contain a transcription termination region downstream of the structural gene to provide for efficient termination. The termination region may be obtained from the same gene as the promoter sequence or may be obtained from different genes. If the mRNA encoded by the structural gene is to be efficiently processed, DNA sequences which direct polyadenylation of the RNA are also commonly added to the vector construct. Polyadenylation sequences include but are not limited to the Agrobacterium octopine synthase signal (Gielen et al, EMBO ./ 3:835-846 (1984)) or the nopaline synthase signal (Depicker et al, Mol. and Appl. Genet. 1:561-573 (1982)). The resulting expression unit is ligated into or otherwise constructed to be included in a vector that is appropriate for higher plant transformation. One or more expression units may be included in the same vector. The vector will typically contain a selectable marker gene expression unit by which transformed plant cells can be identified in culture. Usually, the marker gene will encode resistance to an antibiotic, such as G418, hygromycin, bleomycin, kanamycin, or gentamicin or to an herbicide, such as glyphosate (Round-Up) or glufosinate (BASTA) or atrazine. Replication sequences, of bacterial or viral origin, are generally also included to allow the vector to be cloned in a bacterial or phage host; preferably a broad host range for prokaryotic origin of replication is included. A selectable marker for bacteria may also be included to allow selection of bacterial cells bearing the desired construct. Suitable prokaryotic selectable markers include resistance to antibiotics such as ampicillin, kanamycin or tetracycline. Other DNA sequences encoding additional functions may also be present in the vector, as is known in the art. For instance, in the case of Agrobacterium transformations, T-DNA sequences will also be included for subsequent transfer to plant chromosomes.

To introduce a desired gene or set of genes by conventional methods requires a sexual cross between two lines, and then repeated back-crossing between hybrid offspring and one of the parents until a plant with the desired characteristics is obtained. This process, however, is restricted to plants that can sexually hybridize, and genes in addition to the desired gene will be transferred.

Recombinant DNA techniques allow plant researchers to circumvent these limitations by enabling plant geneticists to identify and clone specific genes for desirable traits, such as improved fatty acid composition, and to introduce these genes into already useful varieties of plants. Once the foreign genes have been introduced into a plant, that plant can then be used in imp plant breeding schemes (e.g., pedigree breeding, single-seed-descent breeding schemes, reciprocal recurrent selection) to produce progeny which also contain the gene of interest.

Genes can be introduced in a site directed fashion using homologous recombination. Homologous recombination permits site-specific modifications in endogenous genes and thus inherited or acquired mutations may be corrected, and/or novel alterations may be engineered into the genome. Homologous recombination and site-directed integration in plants are discussed in, for example, U.S. Patent Nos. 5,451,513; 5,501,967 and 5,527,695.

Although less preferred, each of the above described polynucleotide sequences can be separately introduced into a plant cell by using three separate nucleic -acid constructs. In some embodiments, the three polynucleotide sequences can be co-introduced and co expressed in the plant cell using a single nucleic acid construct. Such a construct can be designed with a single promoter sequences co-which can transcribe a polycistronic message including all three polynucleotide sequences. To enable co-translation of the three polypeptides encoded by the polycistronic message, the polynucleotide sequences can be inter-linked via an internal ribosome entry site (IRES) sequence which facilitates translation of polynucleotide sequences positioned downstream of the IRES sequence. In this case, a transcribed polycistronic RNA molecule encoding the three polypeptides described above will be translated from both the capped 5' end and the two internal IRES sequences of the polycistronic RNA molecule to thereby produce in the cell all three polypeptides.

Alternatively, the polynucleotide segments encoding the plurality of polypeptides capable of conferring increased suberin content in plant cells, plant tissues, plant parts and whole plants can be translationally fused via a protease recognition site cleavable by a protease expressed by the cell to be transformed with the nucleic acid construct. In this case, a chimeric polypeptide translated will be cleaved by a cell-expressed protease to thereby generate the plurality of polypeptides.

In **the fifth aspect,** the invention pertains to recombinant plant parts, such as recombinant plant seeds, fruit, rootstock or cutting, comprising a nucleic acid, recombinant expression vector and/or recombinant cell, according to any of the previous aspects of the invention.

In **the sixth aspect,** the invention pertains to a method of enhancing plant growth or yield comprising: providing a recombinant plant transformed with the nucleic acid construct according to any one of the preceding aspects; and growing the plant under conditions effective to permit the nucleic acid construct to express the HSFB2a polypeptide in the recombinant plant, and thereby enhance plant growth or yield; and/or to a method of enhancing plant growth or yield comprising: providing a recombinant plant seed transformed with the nucleic acid construct according to any one of the preceding aspects; planting the recombinant plant seed in a growth medium; and propagating a recombinant plant from the plant seed to permit the nucleic acid construct to express the HSFB2a polypeptide in the recombinant plant, and thereby enhance plant growth or yield; and/or the invention pertains to a method of enhancing plant growth or yield comprising: providing a rootstock, cutting, or seed according any one of the preceding aspects; introducing the rootstock, cutting, or seed into a growth medium; and propagating a recombinant plant from the rootstock, cutting, or seed to permit the nucleic acid construct to express the HSFB2a polypeptide in the recombinant plant, and thereby enhance plant growth or yield.

In **the seventh aspect,** the invention pertains to a method for alleviating stigma exertion in a plant, the method comprising a step of artificially increasing in the plant an amount, level, concentration and/or activity of
(i) a protein having an amino acid sequence shown in SEQ ID NO: 1, or a protein variant having an amino acid sequence with at least 60% sequence identity compared to the sequence shown in SEQ ID NO: 1; and/or
(ii) a nucleic acid, such as a gene or mRNA, encoding a protein or protein variant of (i).

In **the eighth aspect,** the invention pertains to a method for alleviating stigma exertion in a plant, the method comprising a step of artificially decreasing in the plant an amount, level, concentration and/or activity of
(iii) a protein having an amino acid sequence shown in SEQ ID NO: 1, or a protein variant having an amino acid sequence with at least 60% sequence identity compared to the sequence shown in SEQ ID NO: 1; and/or
(iv) a nucleic acid, such as a gene or mRNA, encoding a protein or protein variant of (i).

In this aspect, as in the respective alternatives aspects of previous description relating to a reduction of HSFB2a expression, activity and/or function in a plant or part of a plant, the mutated HSFB2a is a CRISPR/Cas9 (gene editing) induced mutation that for example may lead to a non-functional variant by point mutation, or by introducing a premature stop codon into the gene of HSFB2a. The person of skill can develop a HSFB2a protein (or gene for expression of mutated version thereof) that has either a reduced expression ( for example by mutating a regulatory element) or has reduced activity by mutating the open reading frame of the HSFB2a protein and thereby introducing a mutation into the HSFB2a protein sequence. A preferred version is the mutated version of tomato HSFB2a as disclosed herein in the example section below.

The use of such a mutated version and plants comprising it is that Stigma exertion is an essential outcrossing trait that can improve hybrid seed production efficiencies.

In view of the above, it will be appreciated that the present invention also relates to the following itemised embodiments:
Item 1: A nucleic acid construct comprising (i) a polynucleotide encoding a heat stress transcription factor B2a (HSFB2a) protein or (ii) polynucleotide encoding a mutated or inactivated heat stress transcription factor B2a (HSFB2a) protein; and a heterologous, constitutive- or tissue-specific promoter operably linked to the polynucleotide encoding the HSFB2a polypeptide, wherein the promoter specifically directs expression of the HSFB2a polypeptide in the plant or tissue of the plant.
Item 2: The nucleic acid construct of item 1, wherein the nucleic acid construct further comprises a 3' transcription termination polynucleotide.
Item 3: The nucleic acid construct of item 1 or 2, wherein the nucleic acid construct comprises DNA.
Item 4: The nucleic acid construct of any one of items 1 to 3, wherein the promoter is a constitutive expression promoter, such as Cauliflower Mosaic Virus 35S (PCaMV35S) promoter.
Item 5: The nucleic acid construct of any one of items 1 to 4, comprising CaMV35S terminator sequence (TCaMV35S).
Item 6: The nucleic acid construct of any one of items 1 to 5, comprising a sequence shown in SEQ ID NO: 3 (3x HA sequence in Invention Disclosure), or in case of (ii) comprising a sequence according to SEQ ID NO: 4 (mutated sequence).
Item 7: A recombinant expression vector comprising the nucleic acid construct according to any one of items 1 to 6.
Item 8: The recombinant expression vector according to item 7, wherein the vector is operable in dicots, preferably in a tomato plant.
Item 9: The recombinant expression vector according to item 7 or 8, wherein the vector is a plasmid.
Item 10: The recombinant expression vector according to any one of items 7 to 9, wherein the vector is *Agrobacterium tumefaciens* strain comprising the plasmid.
Item 11: A recombinant host cell comprising the nucleic acid construct of any of any one of items 1 to 6 or the recombinant expression vector according to any one of items 7 to 10.
Item 12: The recombinant host cell according to item 11, wherein the host cell is a plant cell.
Item 13: The recombinant host cell according to item 11, wherein the host cell is a bacterium.
Item 14: A recombinant plant or plant part, comprising a genetic modification which (i) enhances the expression of heat stress transcription factor B2a in a tissue of the plant; or (ii) which reduces or decreases the expression of heat stress transcription factor B2a in a tissue of the plant.
Item 15: The recombinant plant or plant part of item 14, comprising a heterologous sequence for the ectopic expression of HSFB2a, or a variant thereof, wherein the variant has an amino acid sequence that is at least 80% identical to SEQ ID NO: 1 (AS of HSFB2a).
Item 16: The recombinant plant or plant part of item 14 or 15, wherein the plant genome carries an artificially modified genomic mutant of an endogenous HSFB2a gene which compared to the wild type gene sequence of said plant has an in the case of (i) an increased expression of HSFB2a in a tissue of the plant, or (ii) has a decreased expression of HSFB2a in a tissue of the plant.
Item 17: The recombinant plant or plant part of item 16, wherein the mutant is a genetic mutation of a coding sequence of the HSFB2a gene or of a regulator sequence of the HSFB2a gene.
Item 18: The recombinant plant or plant part of item 17, wherein the plant is a tomato plant, preferably from the line *Moneymaker.*
Item 19: The recombinant plant or plant part of item 17 comprising a nucleic acid construct of any one of items 1 to 6.
Item 20: The recombinant plant or plant part of any one of items 14 to 19, comprising a recombinant host cell of any one of items 11 to 13.
Item 21: The recombinant plant or plant part, wherein the plant part is a rootstock, cutting, seed or fruit.
Item 22: A recombinant plant seed comprising a recombinant host cell according to item 11 to 13.
Item 23: A recombinant plant seed comprising the nucleic acid construct according to any one of items 1 to 6.
Item 24: The recombinant plant seed of item 22 or 23, wherein the recombinant plant is a tomato, preferably *Solanum lycopersicum.*
Item 25: A rootstock, cutting, fruit or seed obtained from the recombinant plant according to any one of the preceding items.
Item 26: A method of enhancing plant growth or yield comprising: providing a recombinant plant transformed with the nucleic acid construct according to any one of items 1 to 6; and growing the plant under conditions effective to permit the nucleic acid construct to express the HSFB2a polypeptide in the recombinant plant, and thereby enhance plant growth or yield.
Item 27: A method of enhancing plant growth or yield comprising: providing a recombinant plant seed transformed with the nucleic acid construct according to any one of items 1 to 6; planting the recombinant plant seed in a growth medium; and propagating a recombinant plant from the plant seed to permit the nucleic acid construct to express the HSFB2a polypeptide in the recombinant plant, and thereby enhance plant growth or yield.
Item 28: A method of enhancing plant growth or yield comprising: providing a rootstock, cutting, or seed according to item 25; introducing the rootstock, cutting, or seed into a growth medium; and propagating a recombinant plant from the rootstock, cutting, or seed to permit the nucleic acid construct to express the HSFB2a polypeptide in the recombinant plant, and thereby enhance plant growth or yield.
Item 29: The method of any one of items 26 to 28, wherein the enhanced plant growth or yield is in comparison to a non- recombinant (wild type) plant of the same variety.
Item 30: The method of any one of items 26 to 28, wherein the recombinant plant is grown under conditions comprising heat stress.
Item 31: The method of any one of items 26 to 30, wherein the enhanced plant growth or yield is selected from:
   (i) faster vegetative growth,
   (ii) increased biomass yields,
   (iii) enhanced root growth,
   (iv) increased seed/grain production,
   (v) improved nutrient contents in biomass,
   (vi) increased fruit production, and
   any combinations thereof.
Item 32: A method for alleviating stigma exertion in a plant, the method comprising a step of artificially increasing in the plant an amount, level, concentration and/or activity of
   (i)a protein having an amino acid sequence shown in SEQ ID NO: 1, or a protein variant having an amino acid sequence with at least 60% sequence identity compared to the sequence shown in SEQ ID NO: 1; and/or
   (ii)a nucleic acid, such as a gene or mRNA, encoding a protein or protein variant of (i).
Item 33: The method of item 32, wherein the stigma exertion is a heat-induced stigma exertion.
Item 34: A method for promoting or increasing stigma exertion in a plant, the method comprising a step of artificially decreasing in the plant an amount, level, concentration and/or activity of
   (v)a protein having an amino acid sequence shown in SEQ ID NO: 1, or a protein variant having an amino acid sequence with at least 60% sequence identity compared to the sequence shown in SEQ ID NO: 1; and/or
   (vi)a nucleic acid, such as a gene or mRNA, encoding a protein or protein variant of (i).
Item 35: The method of claim 34, wherein the stigma exertion is a heat-induced stigma exertion.

The terms "of the [present] invention", "in accordance with the invention", "according to the invention" and the like, as used herein are intended to refer to all aspects and embodiments of the invention described and/or claimed herein.

As used herein, the term "comprising" is to be construed as encompassing both "including" and "consisting of", both meanings being specifically intended, and hence individually disclosed embodiments in accordance with the present invention. Where used herein, "and/or" is to be taken as specific disclosure of each of the two specified features or components with or without the other. For example, "A and/or B" is to be taken as specific disclosure of each of (i) A, (ii) B and (iii) A and B, just as if each is set out individually herein. In the context of the present invention, the terms "about" and "approximately" denote an interval of accuracy that the person skilled in the art will understand to still ensure the technical effect of the feature in question. The term typically indicates deviation from the indicated numerical value by ±20%, ±15%, ±10%, and for example ±5%. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. Where an indefinite or definite article is used when referring to a singular noun, e.g. "a", "an" or "the", this includes a plural of that noun unless something else is specifically stated.

It is to be understood that application of the teachings of the present invention to a specific problem or environment, and the inclusion of variations of the present invention or additional features thereto (such as further aspects and embodiments), will be within the capabilities of one having ordinary skill in the art in light of the teachings contained herein.

Unless context dictates otherwise, the descriptions and definitions of the features set out above are not limited to any particular aspect or embodiment of the invention and apply equally to all aspects and embodiments which are described.

All references, patents, and publications cited herein are hereby incorporated by reference in their entirety.

### BRIEF DESCRIPTION OF THE FIGURES AND SEQUENCES

The figures show:

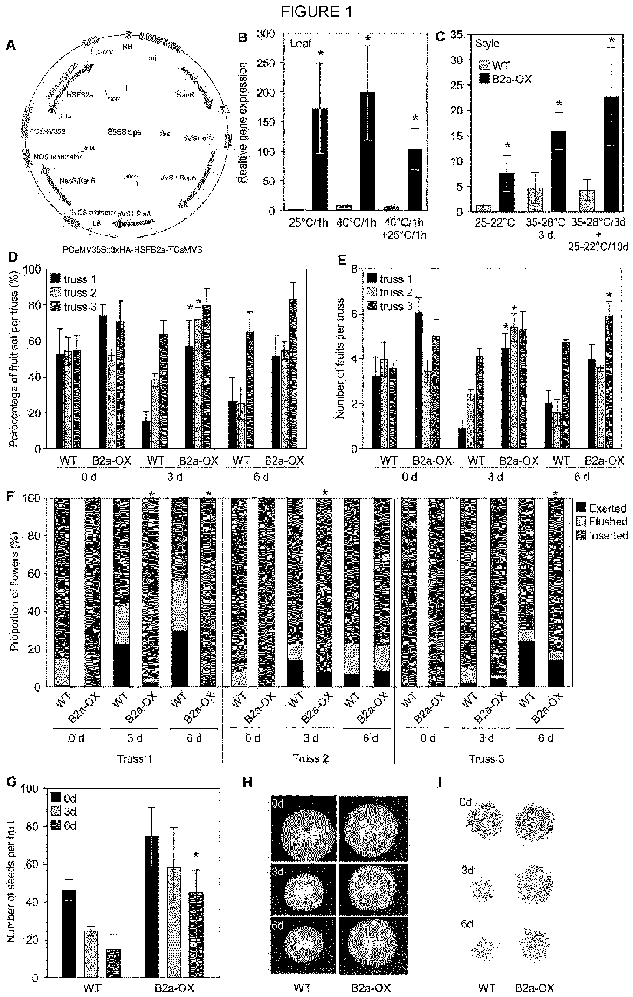

**Figure 1****: shows the ectopic expression of tomato HSFB2a in tomato cultivar Moneymaker increases the frequence of inserted stigmas and the production of fruits in heat stressed plants.** (A) Vector carrying the PCaMV35S::3xHA-HSFB2a cassette used for Agrobacterium-mediated transformation. (B) Total transcript levels of HSFB2a (endogenous and transgene) in tomato leaves from WT (wild type) and B2a-OX (transgenic line) plants exposed to heat stress (1 hour / 40°C) and then allowed to recover (1 hour / 25°C) or kept for 2 hours at 25°C as control. (C) Total HSFB2a transcript levels (endogenous and transgene) in styles of flowers from plants exposed to the indicated temperature regimes. For (B) and (C) the analysis was done by quantitative real time PCR. Transcript levels are expressed relative to WT control for each tissue/organ. (D) Percentage of fruit set, (E) number of fruits per truss, and (F) number of seeds per fruit in WT and B2a-OX plants exposed to the indicated conditions. (H) Representative fruits and (I) seeds from 10 fruits from the same genotypes and conditions. In all cases results are mean of 3-4 independent experiments with 3-4 individual plants each in randomized experiments. Error bars are standard error and asterisk indicates a p-value < 0.05 based on a T-test between the respective B2a-OX and WT samples.

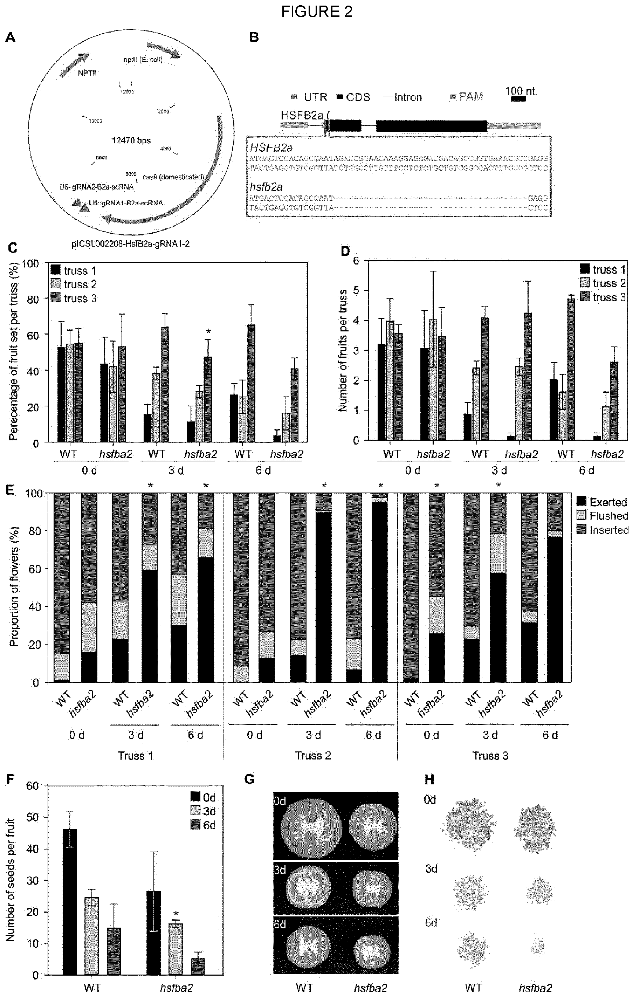

**Figure 2****: shows mutation of tomato HSFB2a in tomato cultivar Moneymaker causes stigma exertion and lower production of fruits and seeds in heat stressed plants.** (A) Vector carrying the expression cassettes for CRISPR/Cas9 mediated gene editing used to generated the mutant *hsfb2a* line. (B) Gene structure of HSFB2a, region targeted for gene editing, and the resulted mutation (deletion of 35 nucleotides). PAM sequences targeted by two independent gRNAs are shown in red color. (C) Percentage of fruit set, (D) number of fruits per truss, and (E) number of seeds per fruit in WT and *hsfb2a* plants exposed to the indicated conditions. (F) Representative fruits and (G) seeds from 10 fruits from the same genotypes and conditions. In all cases results are mean of 3-4 independent experiments with 3-4 individual plants each in randomized experiments. Error bars are standard error and asterisk indicates a p-value < 0.05 based on a T-test between the respective *hsfb2a* and WT samples.

The sequences show:
**SEQ ID NO. 1** HSFB2a (WT) protein sequence: 
MTPQPIDRNKGETTAGETPRSVPTPFLTKTYQLIEDQSIDDVISWNEDGSTFIVWNPTEF
ARDLLPKYFKHNNFSSFVRQLNTYGFRKVVPDRWEFANDSFRRGERSQLVDIQRRKVATPIATP
SAAATAVAIVASPPQPPPPPPSPSPPPPPPAQPPVAVSTSDSCEEQVLSSNSSAGSTAELLGEN
ERLRLENLQLSKELNQMKKLCGNIYGMMSTYAHPSSSGNQSAESSSPSLKPLDLLGTERSVGE
SQVKAVEDRESHEVPEEAQARLFGFSIGMKRVREGEQAMMEHCHDLRLRQPGTTDVKVEPSD
QESNGESEERSWLLHCGGRNQRTCN
**SEQ ID NO. 2** 3xHA-HSFB2a protein sequence: 
MTPQPIDRNKGETTAGETPRSVPTPFLTKTYQLIEDQSIDDVISWNEDGSTFIVWNPTEF
ARDLLPKYFKHNNFSSFVRQLNTYGFRKVVPDRWEFANDSFRRGERSQLVDIQRRKVATPIATP
SAAATAVAIVASPPQPPPPPPSPSPPPPPPAQPPVAVSTSDSCEEQVLSSNSSAGSTAELLGEN
ERLRLENLQLSKELNQMKKLCGNIYGMMSTYAHPSSSGNQSAESSSPSLKPLDLLGTERSVGE
SQVKAVEDRESHEVPEEAQARLFGFSIGMKRVREGEQAMMEHCHDLRLRQPGTTDVKVEPSD
QESNGESEERSWLLHCGGRNQRTCN
**SEQ ID NO. 3** HSFB2a nucleic acid coding sequence: 
ATGACTCCACAGCCAATAGACCGGAACAAAGGAGAGACGACAGCCGGTGAAACGC
CGAGGTCTGTGCCGACACCGTTTTTAACAAAGACCTACCAGCTCATCGAGGATCAATCTATT
GACGATGTGATCTCTTGGAATGAAGACGGATCTACTTTTATAGTGTGGAATCCAACGGAGTT
CGCTAGGGATTTGCTTCCTAAATATTTTAAACATAATAATTTTTCTAGCTTCGTTAGGCAGCTC
AACACCTATGGATTTCGGAAAGTAGTACCTGATCGATGGGAATTCGCAAACGACAGCTTCCG
GAGAGGTGAGAGAAGTCAGTTAGTTGATATCCAGCGTCGAAAGGTTGCCACGCCGATTGCT
ACTCCTTCCGCAGCTGCAACAGCGGTGGCGATTGTGGCTTCTCCGCCTCAGCCTCCACCT
CCACCTCCGTCTCCGTCTCCGCCTCCGCCTCCGCCTGCTCAACCACCGGTGGCTGTATCT
ACCTCTGATTCCTGTGAAGAGCAAGTTCTATCGTCGAACTCATCTGCAGGTAGCACAGCGG
AGCTTTTAGGAGAAAACGAACGGTTAAGATTGGAGAATTTACAGCTCAGCAAAGAGTTGAAC
CAAATGAAGAAGCTCTGCGGCAATATATATGGTATGATGTCGACTTACGCACATCCTTCGAG
CAGCGGTAATCAATCGGCGGAGAGTAGTTCGCCTTCGTTGAAGCCACTGGATCTGTTAGGG
ACGGAACGGTCCGTAGGTGAATCTCAGGTGAAGGCGGTAGAGGACCGGGAGAGCCATGA
GGTACCGGAGGAAGCGCAGGCGAGATTATTCGGTTTCTCGATAGGCATGAAGCGTGTTAGG
GAAGGGGAACAAGCAATGATGGAGCATTGTCATGATTTACGGCTGCGGCAACCTGGAACAA
 
CGGATGTTAAAGTAGAACCGTCTGATCAGGAAAGTAACGGTGAAAGTGAAGAGAGATCGTG
GCTGCTACATTGCGGTGGAAGAAATCAAAGGACTTGTAATTGATAG
 (underlined bold sequence indicate the section deleted in the mutant construct)
**SEQ ID NO. 4** 3xHA-HSFB2a nucleic acid coding sequence: 
ATGGTCTTTTACCCATACGATGTTCCTGACTATGCGGGCTATCCCTATGACGTCCCG
GACTATGCAGGATCCTATCCATATGACGTTCCAGATTACGCTGCTCAGGTACCGTCGAGTAC
TCCACAGCCAATAGACCGGAACAAAGGAGAGACGACAGCCGGTGAAACGCCGAGGTCTGT
GCCGACACCGTTTTTAACAAAGACCTACCAGCTCATCGAGGATCAATCTATTGACGATGTGA
TCTCTTGGAATGAAGACGGATCTACTTTTATAGTGTGGAATCCAACGGAGTTCGCTAGGGAT
TTGCTTCCTAAATATTTTAAACATAATAATTTTTCTAGCTTCGTTAGGCAGCTCAACACCTATG
GATTTCGGAAAGTAGTACCTGATCGATGGGAATTCGCAAACGACAGCTTCCGGAGAGGTGA
GAGAAGTCAGTTAGTTGATATCCAGCGTCGAAAGGTTGCCACGCCGATTGCTACTCCTTCC
GCAGCTGCAACAGCGGTGGCGATTGTGGCTTCTCCGCCTCAGCCTCCACCTCCACCTCCG
TCTCCGTCTCCGCCTCCGCCTCCGCCTGCTCAACCACCGGTGGCTGTATCTACCTCTGATT
CCTGTGAAGAGCAAGTTCTATCGTCGAACTCATCTGCAGGTAGCACAGCGGAGCTTTTAGG
AGAAAACGAACGGTTAAGATTGGAGAATTTACAGCTCAGCAAAGAGTTGAACCAAATGAAGA
AGCTCTGCGGCAATATATATGGTATGATGTCGACTTACGCACATCCTTCGAGCAGCGGTAAT
CAATCGGCGGAGAGTAGTTCGCCTTCGTTGAAGCCACTGGATCTGTTAGGGACGGAACGG
TCCGTAGGTGAATCTCAGGTGAAGGCGGTAGAGGACCGGGAGAGCCATGAGGTACCGGA
GGAAGCGCAGGCGAGATTATTCGGTTTCTCGATAGGCATGAAGCGTGTTAGGGAAGGGGA
ACAAGCAATGATGGAGCATTGTCATGATTTACGGCTGCGGCAACCTGGAACAACGGATGTT
AAAGTAGAACCGTCTGATCAGGAAAGTAACGGTGAAAGTGAAGAGAGATCGTGGCTGCTAC
ATTGCGGTGGAAGAAATCAAAGGACTTGTAATTGA
SEQ **ID NO:** 5 top sequence shown in Figure 2B
   ATGACTCCACAGCCAATAGACCGGAACAAAGGAGAGACGACAGCCGGTGAAACGC CGA
**SEQ ID NO: 6** bottom sequence shown in Figure 2B
   ATGACTCCACAGCCAATGAGG
**SEQ ID NO: 7 to 12:** primer and probe sequences (see examples below).

### EXAMPLES

Certain aspects and embodiments of the invention will now be illustrated by way of example and with reference to the description, figures and tables set out herein. Such examples of the methods, uses and other aspects of the present invention are representative only, and should not be taken to limit the scope of the present invention to only such representative examples.

The invention provides transgenic tomato (*Solanum lycopersicum* cv. Moneymaker) line that constitutively and ectopically expresses the *Solanum lycopersicum* gene heat stress transcription factor B2a (HSFB2a; gene identification number SOLYC08G080540). HSFB2a is a heat stress induced gene, both in vegetative and reproductive tissues as styles. The ectopic expression of HSFB2a in the transgenic line (B2a-OX) leads to a higher proportion of flowers with an inserted stigma after 3 or 6 days of heat stress compared to wild-type plants. As a result, the B2a-OX line demonstrates increased fruit set, higher fruit production, and an increased number of seeds per fruit under heat stress conditions.

The examples show:

### Example 1: Generation of HSFB2a expression transgenic tomato line

Dsd The coding sequence of HSFB2a (SOLYC08G080540; grey highlighted nucleotide and amino acid sequence) from Solanum lycopersicum var. Moneymaker was cloned in a pRT vector carrying two consecutive copies of the Cauliflower Mosaic Virus 35S (PCaMV35S) promoter and the CaMV35S terminator sequence (TCaMV35S). HSFB2a was fused at the 5'-prime with tripartide human influenza hemagglutinin coding sequence (HA) coding sequence followed by a -AQVPSS- linker. The PCaMV35S::3xHA-HSFB2a:: (TCaMV35S) was amplified by polymerase chain reaction using the oligonucleotides that harbor Bsal endonuclease recognisition sites (Pf: 5'-TAGGTCTCTGGAGTAGGCTTTACACTTTATGC-3'(SEQ ID NO: 7),

Pr: 5'-TACGGTCTCTAGCGTACGGTCACAGCTTGTCTG-3'(SEQ ID NO: 8)). The PCR fragment was subcloned into the pICH86966 binary vector (Reference (Weber *et al.,* 2011 generated by Sylvestre Marillonnet (Addgene plasmid # 48075 ; http://n2t.net/addgene:48075 ; RRID:Addgene_48075) by Golden Gate cloning as described by Weber *et al.*, (2011). plCH86966 carries the neomycin phosphotransferase II (NPTII) expression cassette for kanamycin-based selection.

The final vector (Figure 1A) was transformed into Agrobacterium tumefaciens strain GV3101 which was used for transformation of *Solanum lycopersicum* cv. Moneymaker cotyledons as described previously (McCormick *et al.,* 1986). Transgenic plants were selected based on kanamycin resistance and presence of the transgene by PCR.

For gene expression analysis, young leaves were collected from six-week old plants exposed to 40°C for 1 hour (heat stress: HS sample) in plant growth chambers (CLF PlantClimatics GmbH), allowed to recover for 1 hour at 25°C (recovery: R sample), or kept for 2 hours at 25°C as controls (C sample). Ectopic expression of HSFB2a was confirmed by quantitative real time PCR (qRT-PCR), using specific oligonucleotides (oligonucleotide forward: GGAAAGTAGTACCTGATCGATGGG (SEQ ID NO: 9), oligonucleotide reverse: CAGAGGTAGATACAGCCACCGG (SEQ ID NO: 10)) and the analysis was done as previously described (Mesihovic *et al.,* 2022).

### Example 2: Stress treatment and thermotolerance

WT and B2a-OX young seedlings were planted in soil supplemented with perlite and allowed to grow in a glasshouse under 120 µmol m-2 s-1 light intensity for 16 hours at 25°C and 8 hours darkness at 22°C. Plants at the stage when the first flower of the oldest inflorescence (truss 1) reached anthesis were transferred to controlled plant chambers (CLF PlantClimatics GmbH) and exposed for 3 or 6 days to 35°C day (16 hours 6.00 am - 22.00 pm) / 28°C night (6 hours 22.00 pm - 6.00 am) treatment, with 30 min gradual increase and decrease of the temperature at beginning and end of the day. Control plants were kept in chambers at 25°C day / 22°C night cycle. Humidity at all cases was approximately 70% and light intensity 120 µmol m-2 s-1.

At the begin of the treatment, the buds on the second inflorescence were at very young stage (up to 4 mm bud length) and the buds of the 3rd inflorescence had not been formed in any genotype.

Following the treatments plants were returned to the greenhouse under the conditions described above. Thermotolerance was assessed based on attributes related to yield. Overall, the transgenic line B2a-OX showed enhanced fruit set for the first and second truss in plants exposed to 3 days of heat stress and in all three examined trusses in plants exposed to 6 days compared to control plants. B2a-OX plants exposed to 3 or 6 days of heat stress produced a higher number of fruits compared to WT plants exposed to the same conditions. In addition, the B2a-OX fruits produce more seeds than the WT plants after four days of heat stress.

### Example 3: Generation of hsfb2a mutant lines by CRISPR/Cas9 gene editing

For the CRISPR / Cas9-mediated mutation of *Solanum lycopersicum* HSFB2a gene (SOLYC08G080540), two sgRNAs targeting each gene were selected (gRNA1: CGACAGCCGGTGAAACGCCG (SEQ ID NO: 11); gRNA2: TGACTCCACAGCCAATAGAC (SEQ ID NO: 12)) recognizing PAM sequences in the - strand using CRISPR-PLANT (Minkenberg *et al.,* 2019). The assembly was done using the Golden Gate cloning method (Engler *et al.,* 2014). *Arabidopsis thaliana* U6 promoter was cloned from the plasmid pICSL01009:AtU6p (Addgene under #46968), used to 1to create AtU6p::gRNA1-scRNA and AtU6p::gRNA2-scRNA modules that were cloned initially in Level 1 vectors plCH47732 and pICH47742, respectively, and then along with the end-Linker containing plasmid plCH49266 to the final vector plCSL002208 (Fig. 2A). All Level 1 plasmids were taken from the MoClo Toolkit (Addgene #1000000044) as outlined by Engler et al. (2014). The final plasmid, plCSL002208, generously provided by Dr. Nicola Patron from the Earlham Institute in the UK, containing the *Streptococcus pyogenes* Cas9 gene driven by the CaMV35S promoter, and the kanamycin resistance gene NPTII under the NOS promoter for selection. The vector was introduced into the *Agrobacterium tumefaciens* strain GV3101. *Solanum lycopersicum* cv. Moneymaker cotyledons were used for Agrobacterium mediated transformation based on the protocol of (McCormick *et al.,* 1986). Genotyping by Sanger sequencing was used to confirm the mutation. Experiments were conducted using T2 generation homozygous mutants that are T-DNA free. A mutant line having a 35 nucleotide deletion was obtained and called "*hsfb2a*" (Fig. 2B; See sequence below). The deletion created a premature termination codon, eliminating the possibility for the synthesis of the full length protein.

### Example 4: Stress treatment and thermotolerance

Wild type *Solanum lycopersicum* cv. Moneymaker (WT) and *hsfb2a* young seedlings were planted in soil supplemented with perlite and allowed to grow in a glasshouse under 120 µmol m^{- 2} s-1 light intensity for 16 hours at 25°C and 8 hours darkness at 22°C. Plants at the stage when the first flower of the oldest inflorescence (truss 1) reached anthesis were transferred to controlled plant chambers (CLF PlantClimatics GmbH) and exposed for 3 or 6 days to 35°C day (16 hours 6.00 am - 22.00 pm) / 28°C night (6 hours 22.00 pm - 6.00 am) treatment, with 30 min gradual increase and decrease of the temperature at beginning and end of the day. Control plants were kept in chambers at 25°C day / 22°C night cycle. Humidity at all cases was approximately 70% and light intensity 120 µmol m⁻² s⁻¹. At the begin of the treatment, the buds on the second inflorescence were at very young stage (up to 4 mm bud length) and the buds of the 3^{rd} inflorescence had not been formed in any genotype.

Following the treatments plants were returned to the greenhouse under the conditions described above. Thermotolerance was assessed based on attributes related to yield. Overall the mutant line *hsfb2a* showed reduced fruit set compared to the WT at the 3^{rd} truss, after 3 days of heat stress (Fig. 2C), and lower number of fruits in the first and third truss after 6 days of heat stress (Fig. 2D). Overall *hsfb2a* showed a lower number of flowers with inserted stigma in all three inflorescences (trusses) after 3 days of heat stress when compared to WT, and lower number of flowers with inserted stigmas in the second inflorescence after 6 days of heat stress. The first and second inflorescence of *hsfb2a* plants also exhibited a higher number of flowers with exerted stigma after 3 and 6 days of heat stress, compared to the corresponding WT flowers treated the same way (Fig. 2E). Fruits from *hsfb2a* exposed to 3 days of heat stress produced significantly lower number of seeds compared to WT (Fig. 2F-H). Overall these results show that the mutation of HSFB2a results in stigma exertion in plants exposed to heat stress and this is associated with lower fruit set, reduced number of fruits and reduced seed production per fruit.

## Claims

1. **A nucleic acid construct** comprising (i) a polynucleotide encoding a heat stress transcription factor B2a (HSFB2a) protein or (ii) polynucleotide encoding a mutated or inactivated heat stress transcription factor B2a (HSFB2a) protein; and a heterologous, constitutive- or tissue-specific promoter operably linked to the polynucleotide encoding the HSFB2a polypeptide, wherein the promoter specifically directs expression of the HSFB2a polypeptide in the plant or tissue of the plant.

2. The nucleic acid construct of claim 1, wherein the promoter is a constitutive expression promoter, such as Cauliflower Mosaic Virus 35S (PCaMV35S) promoter.

3. The nucleic acid construct of claim 1 or 2, comprising a sequence shown in SEQ ID NO: 3 (3x HA sequence in Invention Disclosure), or in case of (ii) comprising a sequence according to SEQ ID NO: 4 (mutated sequence).

4. **A recombinant expression vector** comprising the nucleic acid construct according to any one of claims 1 to 3.

5. The recombinant expression vector according to claim 4, wherein the vector is operable in dicots, preferably in a tomato plant.

6. **A recombinant host cell** comprising the nucleic acid construct of any of any one of claims 1 to 3 or the recombinant expression vector according to any one of claims 4 or 5.

7. The recombinant host cell according to claim 6, wherein the host cell is a plant cell or is a bacterium.

8. **A recombinant plant or plant part,** comprising a genetic modification which (i) enhances the expression of heat stress transcription factor B2a in a tissue of the plant; or (ii) which reduces or decreases the expression of heat stress transcription factor B2a in a tissue of the plant.

9. The recombinant plant or plant part of claim 8, comprising a heterologous sequence for the ectopic expression of HSFB2a, or a variant thereof, wherein the variant has an amino acid sequence that is at least 80% identical to SEQ ID NO: 1 (AS of HSFB2a).

10. The recombinant plant or plant part of claim 8 or 9, wherein the plant genome carries an artificially modified genomic mutant of an endogenous HSFB2a gene which compared to the wild type gene sequence of said plant has an in the case of (i) an increased expression of HSFB2a in a tissue of the plant, or (ii) has a decreased expression of HSFB2a in a tissue of the plant.

11. The recombinant plant or plant part of any one of claims 8 to 10, wherein the plant is a tomato plant, preferably from the line *Moneymaker.*

12. **A recombinant plant seed** comprising the nucleic acid construct according to any one of claims 1 to 3 and/or comprising a recombinant host cell according to claim 6 or 7.

13. **A method of enhancing plant growth or yield** comprising: providing a recombinant plant transformed with the nucleic acid construct according to any one of claims 1 to 3; and growing the plant under conditions effective to permit the nucleic acid construct to express the HSFB2a polypeptide in the recombinant plant, and thereby enhance plant growth or yield.

14. The method of claim 13, wherein the enhanced plant growth or yield is selected from:
(i) faster vegetative growth,
(ii) increased biomass yields,
(iii) enhanced root growth,
(iv) increased seed/grain production,
(v) improved nutrient contents in biomass,
(vi) increased fruit production, and
any combinations thereof.

15. **A method for alleviating stigma exertion in a plant,** the method comprising a step of artificially increasing in the plant an amount, level, concentration and/or activity of
(i) a protein having an amino acid sequence shown in SEQ ID NO: 1, or a protein variant having an amino acid sequence with at least 60% sequence identity compared to the sequence shown in SEQ ID NO: 1; and/or
(ii) a nucleic acid, such as a gene or mRNA, encoding a protein or protein variant of (i).

16. **A method for promoting or increasing stigma exertion in a plant,** the method comprising a step of artificially decreasing in the plant an amount, level, concentration and/or activity of
(i) a protein having an amino acid sequence shown in SEQ ID NO: 1, or a protein variant having an amino acid sequence with at least 60% sequence identity compared to the sequence shown in SEQ ID NO: 1; and/or
(ii) a nucleic acid, such as a gene or mRNA, encoding a protein or protein variant of (i).
